# EUROPÄISCHE PATENTANMELDUNG

(11) **EP 0 744 184 A1**
(43) Veröffentlichungstag der Anmeldung: **27.11.1996**
(21) Anmeldenummer: 96104804.8
(22) Anmeldetag: 26.03.1996
(51) Int. Cl.: A61M 16/08, A61M 16/01

(54) **Behälter für ein Gas bei einem Beatmungs/Narkosesystem**

(30) Priorität: 24.05.1995 SE 9501938
(71) Anmelder: Siemens-Elema AB, 171 95 Solna 1 (SE)
(72) Erfinder: Psaros, Georgius, 146 38 Tullinge (SE)

(57) **Zusammenfassung**

Die Erfindung bezieht sich auf einen Behälter für ein Gas bei einem Beatmungs/Narkosesystem, bei dem der Behälter über ein Ventil an einer Gasquelle angeschlossen ist. Der Behälter ist in seinem Volumen veränderbar und wird von einer konstanten Kraft beeinflußt, um dem Gas im Behälter, unabhängig vom Volumen innerhalb vorgegebener Grenzen, einen konstanten Druck beizubringen. Gas mit dem genannten konstanten Druck kann vom Behälter entnommen und ein Beatmungskreis im Beatmungs/Narkosesystem zugeführt werden. Der Behälter ist derart gestaltet, daß er selbsttätig die genannte konstante Kraft erzeugt. Um einen Behälter dieser Art zu erhalten, der verhältnismäßig leicht und in seinem Aufbau stabil und verhältnismäßig einfach und daher in der Herstellung billig ist, wird erfindungsgemäss vorgeschlagen, daß der Behälter (1) wenigstens teilweise aus einem elastischen Material, z.B. Metall oder Kunststoff besteht, wobei die konstante Kraft durch die Elastizität des Materials erzeugt wird.

## Beschreibung

Die Erfindung bezieht sich auf einen Behälter für ein Gas bei einem Beatmungs/Narkosesystem, bei dem der Behälter über ein Ventil an einer Gasquelle angeschlossen ist, wobei der Behälter in seinem Volumen veränderbar ist und von einer konstanten Kraft beeinflußt wird, um dem Gas im Behälter, unabhängig vom Volumen innerhalb vorgegebener Grenzen einen konstanten Druck beizubringen, wobei Gas mit dem genannten konstanten Druck vom Behälter entnommen und ein Beatmungskreis im Beatmungs/Narkosesystem zugeführt werden kann und wobei der Behälter derart gestaltet ist, daß er selbsttätig die genannte konstante Kraft erzeugt.

In den meisten Krankenhäusern wird im allgemeinen Atemgas, d.h. Luft und Sauerstoff für den Betrieb von Beatmungsgeräten von zentralen Hochdrucksystemen erhalten. Da der Gasdruck zum Betrieb von Beatmungsgeräten bei einem Wert von etwa 100 cm Wassersäule liegen soll, ist eine erhebliche Reduzierung des Druckes der vom Hochdrucksystem kommenden Gase erforderlich. Auch bei Verwendung von Gasflaschen als Gasspender für z.B. Lachgas und Halotan bei einem Beatmungs/Narkosegerät muß für eine solche Druckverminderung gesorgt werden.

Der Betriebsdruck desjenigen Gases, das den Patienten, der an den genannten Geräten angeschlossen ist, zugeführt werden soll, soll genügend hoch sein, um einen erwünschten Inspirationsfluß zu geben, wobei der Betriebsdruck, abhängig vom Widerstand in den Zufuhrkreisen und in den Luftwegen des Patienten, teils den Druckabfall und teils die vom Volumen abhängige Drucksteigerung in den Lungen des Patienten überwinden soll. Der Betriebsdruck darf jedoch aus Sicherheitsgründen einen vorbestimmten Druckwert nicht überschreiten und der Druck, der die genannten Wünsche am besten erfüllt, ist ein konstanter Druck.

In der DE 41 07 666 C2 ist ein Gerät beschrieben, mit dessen Hilfe einer Vene eines Patienten Sauerstoff zugeführt werden kann. Das Gerät umfaßt einen Sauerstoffbehälter in Form eines Faltenbalges, der eine obere Deckplatte mit einem solchen Eigengewicht aufweist, daß der Balg selbsttätig zusammengedrückt werden kann. Damit eine notwendige seitliche Steife erhalten wird, ist die Deckplatte über Kugellager mit acht um den Balg angeordneten Führungsstäben verschiebbar verbunden. Dieser Balg ist im Aufbau verhältnismäßig kompliziert und daher in der Herstellung teuer. Der Balg kann auch aufgrund des verhältnismäßig hohen Gewichts der Deckplatte in gewissen Fällen, insbesondere dann, wenn der Balg eine größere Gasmenge beinhalten soll, verhältnismäßig schwer sein.

Durch die US-PS 3 824 902 ist ein weiterer Behälter bekannt, mit dessen Hilfe ein konstanter Gasdruck erzeugt werden kann. Der Behälter, der die Form eines Faltenbalges hat und aus einen weichen Kunststoffmaterial besteht, ist zwischen zwei steifen Platten plaziert, wobei die obere Platte fest im Raum und die untere Platte über einen Arm an einer Welle schwenkbar gelagert ist, die im umgekehrten Sinn des Uhrzeigers geschwenkt wird, um mittels Zugfedern den Behälter zusammen-zupressen. Durch eine geeignete Plazierung der Befestigungspunkte der Federn und durch eine geeignete Dimensionierung der Federn ist es möglich, eine auf den Behälter ausgeübte Kraft zu erhalten, die im Arbeitsbereich immer konstant ist. Die beschriebene Anordnung zum Aufrechterhalten eines konstanten Gasdruckes ist in ihrem Aufbau verhältnismäßig kompliziert und daher in der Herstellung teuer.

In der SE 443 722 (B) ist ein Narkosesystem beschrieben, bestehend aus einem Beatmungskreis, an den ein Patient anschliessbar ist und aus einem druckübertragenden Gasbehälter und aus einem Betriebskreis zur Steuerung des Atemgasflußes im Beatmungskreis. Der druckübertragende Gasbehälter besteht aus einem Faltenbalg, der in einem weiteren Behälter angeordnet ist, einem sog. bag and bottle, wobei das Innere des Balgs an einen Beatmungskreis und der Raum zwischen dem Balg und den Wänden des weiteren Behälters an den Antriebskreis angeschlossen ist. Wenn eine Inspirationsphase eingeleitet werden soll, wird mit Hilfe des Antriebskreises ein Antriebsgas den Raum zwischen dem Balg und dem Behälter zugeführt. Wenn der Druck in diesem Raum höher als der Druck im Balg wird, wird der Balg zusammengepreßt, wobei dem Patienten Atemgas zugeführt wird. Nach der Inspirationsphase folgt eine Exspirationsphase. Während der Exspirationsphase wird das Antriebsgas von dem Raum zwischen dem Balg und dem Behälter herausgelassen und ein relativer Überdruck in dem Beatmungskreis und in dem Balg entsteht. Der Balg fängt dann an zu expandieren und Atemgas kann aus den Lungen des Patienten herausströmen. Dies wird dann bei jedem Atmungszyklus wiederholt. Auf diese Weise kann ein konstanter Gasdruck erhalten werden. Auch eine solche Anordnung ist in ihrem Aufbau und in ihrer Arbeitsweise kompliziert.

Der Erfindung liegt die Aufgabe zugrunde, einen Behälter der eingangs genannten Art zu schaffen, der verhältnismäßig leicht und im Aufbau stabil und verhältnismäßig sehr einfach und dadurch in der Herstellung billig ist.

Diese Aufgabe wird erfindungsgemäss dadurch gelöst, daß der Behälter wenigstens teilweise aus einem elastischen Material, z.B. Metall oder Kunststoff besteht, wobei die konstante Kraft durch die Elastizität des Materials erzeugt wird. Durch einen derartigen Aufbau des Behälters kann dieser verhältnismäßig leicht und sehr einfach gemacht werden. Somit kann eine erhebliche Reduzierung der Herstellungskosten erreicht werden.

In einer vorteilhaften Weiterbildung der Erfindung wird vorgeschlagen, daß der Behälter ein Balg mit mindestens einer gefalteten Wand ist, die mindestens zum Teil aus dem elastischen Material besteht, wobei mindestens dieser Teil der Falten derart vorgeformt ist, daß die Wand selbsttätig danach strebt, sich zusammenzudrücken.

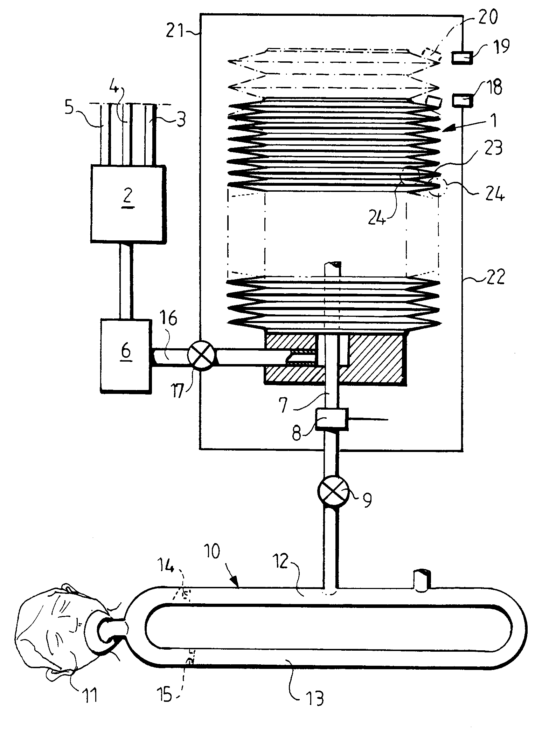

Die Erfindung ist nachfolgend anhand eines in der Zeichnung dargestellten Ausführungsbeispiels näher erläutert.

In der FIG ist ein Gasbehälter 1 nach der Erfindung als ein Teil eines Narkosesystems dargestellt. Das Narkosesystem wird hier lediglich in großen Zügen gezeigt und beschrieben, da dieses Narkosesystem in der schwedischen noch nicht veröffentlichten Patentanmeldung 940 2537-6 näher gezeigt und beschrieben ist. Das Narkosesystem umfaßt u.a. einen Gasmischer 2, der über einen Lachgasanschluß 3 mit Lachgas, über einen Sauerstoffanschluß 4 mit Sauerstoff und über einen Luftanschluß 5 mit Luft versehen werden kann. Während einer Narkose erhält der Patient üblicherweise lediglich eine Mischung von Lachgas und Sauerstoff zusammen mit Narkosegas. Dagegen kann ihm eine Mischung von Luft und Sauerstoff in Verbindung damit, daß der Patient geweckt werden soll, zugeführt werden. Im Gasmischer 2 wird also normalerweise Lachgas und Sauerstoff in vorbestimmten Verhältnissen gemischt und das Gasgemisch wird einem Vergaser 6 zugeleitet. In dem Vergaser 6 kann ein Narkosemittel vergast und der Gasmischung vom Gasmischer 2 zugeleitet werden, bevor sie als frisches Atemgas über eine Gasleitung 16 und über ein erstes Flußventil 17 an den Behälter 1 weitergeleitet wird. Vom Behälter 1 kann dann frisches Atemgas über eine Frischgasleitung 7, durch ein Flußmesser 8 und durch ein zweites Flußventil 9 bis zu einem Beatmungskreis 10 geführt werden. Das Flußventil 9 steuert, abhängig von dem vom Flußmesser 8 gemessenen Fluß, den Fluß mit frischem Atemgas zum Beatmungskreis 10. Um einen möglichst genauen Wert des Flußes zu bekommen, wird der Druck des frischen Atemgases im Behälter 1 so gesteuert, daß er konstant ist. Während einer ersten Zeitperiode wird frisches Atemgas vom Gasmischer 2 über den Vergaser 6 den Behälter 1 zugeführt, um diesen zu füllen. Während einer zweiten Zeitperiode wird durch Schließen des Flußventils 17 der Fluß vom Gasmischer 2 gesperrt und der Behälter 1 wird unter einem beibehaltenen konstanten Druck zum Teil von Frischgas geleert.

Ein Patient 11 ist an den Beatmungskreis 10 angeschlossen. Frisches Atemgas vom Behälter 1 wird einer Inspirationsleitung 12, die das Atmungsgas an den Patienten 11 leitet, zugeführt. Ausgeatmetes Atemgas wird vom Patienten 11 über eine Exspirationsleitung 13 abgeführt. Die Flußrichtung des Atemgases im Beatmungskreis 10 wird mit Hilfe eines Rückschlagventils 14, das in der Inspirationsleitung 12 angebracht ist und mittels eines zweiten Rückschlagventils 15, das in der Exspirationsleitung 13 angeordnet ist, gesteuert.

In dieser FIG ist gezeigt, daß der Behälter 1 ein schlauchförmiger Balg mit einer gefalteten Wand 23 sein kann. In der FIG ist auch gezeigt, daß der Balg 1 zwei vorbestimmte Endlagen aufweist, zwischen denen ein konstanter Gasdruck aufrechterhalten werden kann. Diese Endlagen sind mit Hilfe von Endlageschaltern 18, 19, die im Raum fest angebracht sind, und mittels eines Anzeigers 20, der am Balg 1 befestigt ist, festgelegt. Die Endlageschalter 18, 19 sind über Leitungen 21, 22 an dem Flußventil 17 angeschlossen, wobei das Flußventil 17 schließt, wenn der Anzeiger 20 genau vor dem Endlageschalter 19 steht und öffnet, wenn der Anzeiger 20 genau vor dem Endlageschalter 18 steht. Die strichpunktierten Konturen zeigen an, wenn der Balg 1 in einer maximal herausgezogenen Lage ist, und die durchgezogenen Linien zeigen den Balg 1 in einer maximal zusammengezogenen Lage, innerhalb welcher Lagen der Balg 1 dem Gas im Balg 1 einen konstanten Druck beibringen kann. Der Balg 1 ist derart ausgebildet, daß er selbsttätig die konstante Kraft erzeugt, um dem Gas im Balg 1 diesen konstanten Druck unabhängig vom Volumen innerhalb vorgegebener Grenzen beizubringen. Die faltenförmigen Wände 23 des Balges 1 bestehen aus einem elastischen Material, z.B. Metall oder Kunststoff, wobei die konstante Kraft durch die Elastizität des Materials erzeugt wird. Die in der FIG eingekreisten Falten 24 sind vorzugsweise derart vorgeformt, daß die Wand 23 selbsttätig danach strebt, sich zusammenzudrücken.

Nach der Erfindung kann der Behälter 1 derart ausgebildet sein, daß er gleichzeitig damit, daß er leicht und stabil ist, selbsttätig die konstante Kraft, die dem Gas im Behälter 1 einen konstanten Druck unabhängig vom Volumen innerhalb vorgegebener Grenzen beibringt, erzeugt. Die Kraft soll insbesondere nicht eine von außen beeinflußende Kraft in Form von federgespannten Platten oder in Form eines Gases, das von außen auf dem Behälter drückt, z.B. in der Art bag and bottle, sein. Durch die Erfindung kann ein extrem einfacher und dadurch billiger Behälter hergestellt werden.

Der Behälter 1 nach der Erfindung ist nicht nur darauf begrenzt, in Verbindung mit einem Narkosesystem benutzt zu werden, sondern kann natürlich auch in Verbindung mit Geräten zur reinen Beatmungsunterstützung wie Ventilatoren bzw. Respiratoren verwendet werden.

### Bezugszeichenliste

- 1: Gasbehälter, Balg
- 2: Gasmischer
- 3: Lachgasanschluß
- 4: Sauerstoffanschluß
- 5: Luftanschluß
- 6: Vergaser
- 7: Frischgasleitung
- 8: Flußmesser
- 9,17: Flußventil
- 10: Beatmungskreis
- 11: Patient
- 12: Inspirationsleitung
- 13: Exspirationsleitung
- 14,15: Rückschlagventil
- 16: Gasleitung
- 18,19: Endlageschalter
- 20: Anzeiger
- 21,22: Leitung
- 23: Wand
- 24: Falte

## Patentansprüche

1. Behälter für ein Gas bei einem Beatmungs/Narkosesystem, bei dem der Behälter über ein Ventil an einer Gasquelle angeschlossen ist, wobei der Behälter in seinem Volumen veränderbar ist und von einer konstanten Kraft beeinflußt wird, um dem Gas im Behälter, unabhängig vom Volumen innerhalb vorgegebener Grenzen einen konstanten Druck beizubringen, wobei Gas mit dem genannten konstanten Druck vom Behälter entnommen und ein Beatmungskreis im Beatmungs/Narkosesystem zugeführt werden kann und wobei der Behälter derart gestaltet ist, daß er selbsttätig die genannte konstante Kraft erzeugt, **dadurch gekennzeichnet,** daß der Behälter (1) wenigstens teilweise aus einem elastischen Material, z.B. Metall oder Kunststoff besteht, wobei die konstante Kraft durch die Elastizität des Materials erzeugt wird.

2. Behälter nach Anspruch 1, **dadurch gekennzeichnet,** daß der Behälter ein Balg (1) mit mindestens einer gefalteten Wand (23) ist, die mindestens zum Teil aus dem elastischen Material besteht, wobei mindestens dieser Teil der Falten (24) derart vorgeformt ist, daß die Wand (23) selbsttätig danach strebt, sich zusammenzudrücken.
